# FASCICULE DE BREVET EUROPEEN

(11) **EP 0 624 363 B1**
(45) Date de publication et mention de la délivrance du brevet: **04.08.1999**
(21) Numéro de dépôt: 93108201.0
(22) Date de dépôt: 19.05.1993
(51) Int. Cl.: A61K 7/48

(54) **Composition lipidique pour produits cosmétiques contenant des triglycérides d'acides gras insaturés**
Ungesättigte Fettsäure-Triglyceride enthaltende Lipidzusammensetzung für Kosmetik
Lipid composition for cosmetic products containing unsaturated fatty acid triglycerides

(30) Priorité: 10.05.1993 EP 93107589
(43) Date de publication de la demande: 17.11.1994
(73) Titulaire: SOCIETE DES PRODUITS NESTLE S.A., 1800 Vevey (CH)
(72) Inventeur: Bracco, Umberto, CH-1800 Vevey (CH)
(74) Mandataire: Archambault, Jean

(56) Documents cités:
- FR-A- 2 604 624
- FR-A- 2 648 347
- FR-A- 2 670 111
- FR-A- 2 681 783
- US-A- 4 454 159
- PATENT ABSTRACTS OF JAPAN vol. 12, no. 050 (C-476) 16 Février 1988 & JP-A-62 198 610 (SHISEIDO CO LTD) 2 Septembre 1987

## Description

La présente invention a trait à une composition lipidique destinée à être utilisée dans des compositions cosmétiques.

Les lipides jouent plusieurs rôles en cosmétique:
- au niveau de l'épiderme, comme éléments de structure des membranes cellulaires et de barrière dans le but d'éviter la perte insensible d'eau et donc le désséchement et
- au niveau intra-dermique comme producteurs de substances bio-actives.

Ces substances bio-actives, produits de la transformation enzymatique des acides gras essentiels, sont responsables de réponses physiologiques intéressant la peau, comme l'inflammation, l'hyperprolifération et les mécanismes d'échanges intercellulaires au niveau membranaire. On sait que les lipides, en particulier les phospholipides incorporant les acides gras essentiels, sont les constituants principaux des membranes et règlent leur activité enzymatique, les récepteurs de signaux intercellulaires (les messagers secondaires) et la production des éicosanoïdes.

Le but de l'invention est de fournir une composition lipidique d'application topique basée sur une combinaison d'huiles élaborée pour garantir les qualités structurales et la performance physiologique optimales, avec un équilibre optimum de substances actives afin d'éviter des charges métaboliques déséquilibrées au niveau des tissus, en particulier de la peau.

On connaît, par exemple selon FR-A-2 670 111, une composition de corps gras pour produits cosmétiques dont les triglycérides comportent des acides gras saturés en proportion importante et des acides gras insaturés en moindre proportion. Dans ces conditions, il y a un risque de dissolution partielle des huiles insaturées dans le mélange final et de séparation de glycérides insolubles (saturés) lors de la préparation, le stockage ou l'application du produit.
Par ailleurs, il n'est pas tenu compte d'un équilibre entre les substances actives des familles n-3 et n-6, de l'isomerie des acides gras linoléniques resp. alpha (de la famille n-3) et gamma (de la famille n-6) et de leur concurrence vis-à-vis des enzymes du type des lipoxygenases qui élaborent à partir de ces acides gras des métabolites actifs intervenant dans les processus p.ex. de pro- et anti-inflammation.

La composition lipidique selon l'invention est caractérisée par le fait que les acides gras des triglycérides comprennent, en poids, 40 % à 70 % d'acide oléique et 30 à 50 % d'acides gras polyinsaturés, que le rapport pondéral des acides gras de la famille n-6 sur ceux de la famille n-3 est 10:1 à 30:1 et que les acides gras polyinsaturés comprennent une quantité efficace d'acides des familles n-3 et n-6 de degré d'insaturation 3 et plus.

La composition lipidique d'application topique selon l'invention tient compte non seulement des activités des acides gras essentiels, mais aussi de l'interaction de chaque acide gras essentiel de la famille n-6, par exemple l'acide linoléique, avec son homologue de la famille n-3, par exemple l'acide alpha-linolénique, de l'impossibilité de la peau de transformer ces acides gras en dérivés désaturés supérieurs en C 20 en raison de son incapacité enzymatique à désaturer ces acides, ainsi que de l'activité lipoxygénase de la peau et de la réactivité des hydroxyacides produits par cette activité.

La composition contient une huile riche en acide oléique qui a une action de sructure et de véhicule des acides gras bio-actifs essentiels tout en étant neutre du point de vue de la bioactivité. La teneur élevée en acide oléique confère au mélange lipidique une bonne stabilité à l'oxydation et à la photo-oxydation, ce qui évite la formation de radicaux oxygénés actifs.

Les huiles de choix répondant à cette exigeance sont de préférence, l'huile d'olive, les hybrides de tournesol et de carthame à haute teneur en acide oléique, par exemple > à 70% en poids. On peut aussi citer les oléines d'huiles végétales, par exemple de palme, ou animales, par exemple de lard ou de premier jus, obtenues par fractionnement à sec, par solvant ou par tensioactifs d'huiles et de graisses végétales ou animales.

L'huile en question constitue 40 à 70 %, par exemple environ 60 % en poids du mélange lipidique final.

La composition contient des huiles apportant les acides gras essentiels des familles n-6 et n-3 dans un rapport tenant compte de la plus grande réactivité de ceux de la famille n-3.

Les huiles riches en acide gras de la famille n-6 sont choisies parmi celles riches en acide linoléique, de préférence contenant plus de 65 % en poids de cet acide par rapport aux acides gras totaux, par exemple l'huile de tournesol ou l'huile de pépins de raisin. Parmi ces huiles, la composition comprend celles susceptibles de fournir une quantité efficace d'acide de degré d'insaturation au moins 3, par exemple d'acide gamma-linolénique dont le rôle est de pallier à l'absence des désaturases de la peau. On peut citer l'huile d'onagre, de bourrache et de préférence l'huile de pépins de cassis.

Comme huiles fournissant les acides gras de la famille n-3, on peut citer celles qui contiennent de préférence plus de 35 % en poids d'acide alpha-linolénique par rapport aux acides gras totaux, par exemple l'huile de chia, l'huile de lin ou de préférence l'huile de rosa mosqueta.

Ces acides gras "actifs" peuvent aussi être incorporés à la formulation sous forme d'acides gras libres et/ou de leurs esters d'alcools primaires, en quantités calculées de façon à obtenir les taux et les proportions relatives souhaitées.

La composition moyenne des acides gras des triglycérides de la composition finale est la suivante:

| **Acide gras** | **% en poids** | | **% en poids** |
|---|---|---|---|
| C16:0 | 3-8, | de préférence | 5 |
| C18:0 | 2-6 | " | 4 |
| C18:1 | 45-60 | " | 50 |
| C18:2 | 25-45 | " | 36 |
| C18:3, n-6 (gamma) | 0,2-1 | " | 0,5 |
| C18:3, n-3 (alpha) | 1-5 | " | 3,4 |
| C18:4, n-3 | 0,1-0,5 | " | 0,1 |
| > C20 | 1-3 | " | 1 |

Sur la base de leur compositions respectives en acides gras, les mélanges des huiles ci-après sont préférés:

| **Huile** | **% en poids** | | **% en poids** |
|---|---|---|---|
| Huile de tournesol ou de carthame hybride, riche en acide oléique | 50-70, | de préférence | 60 |
| Huile de tournesol ou de pépins de raisins | 20-40 | " | 31 |
| Huile de rosa mosqueta, de chia ou de lin | 3-10 | " | 6 |
| Huile de pépins de cassis | 1-10 | " | 3 |

Pour pallier à un déficit éventuel du mélange d'huiles en acide gras C20 n-3, on peut l'enrichir par exemple en éthylester d'acide éicosapentaénoïque en quantité suffisante, par exemple à raison d'environ 0,3 % en poids.

La composition selon l'invention peut encore contenir d'autres huiles en quantité mineure, dans le but, par exemple d'améliorer ses propriétés de conservation, par exemple une huile de germes de céréales riche en vitamine E comme antioxydant ou ses propriétés organoleptiques, par exemple l'huile d'abricot.

Elle peut en outre contenir des antioxydants liposolubles ou rendus liposolubles, par exemple un mélange d'acide ascorbique, de lécithine, de tocophérol et de vitamine B.

La composition lipidique selon l'invention est avantageusement utilisée dans diverses compositions cosmétiques pour le traitement de la peau aqueuses ou anhydres, notamment dans des compositions aqueuses comme les fluides, crèmes et laits pour le visage, les mains et le corps, le crèmes et laits anti-solaires, les crèmes et les laits anti-rides et les compositions similaires.

La composition cosmétique en question peut se présenter notamment sous la forme de solution, d'émulsion eau-dans-l'huile ou huile-dans-l'eau, de suspension ou d'aérosol.

Comme compositions cosmétiques anhydres incorporant la composition lipidique selon l'invention, on peut citer les huiles pour le corps, les baumes anhydres, les huiles anti-solaires et les rouges à lèvres.

Dans une telle composition cosmétique, la composition lipidique selon l'invention peut représenter 1 à 80%, et de préférence 5 à 60 % en poids.

Une telle composition cosmétique renferme généralement, en quantités appropriées, des adjuvants tels que, par exemple les émulsifiants, les agents anti-transpirants, les stabilisants, les conservateurs, les filtres solaires, les parfums, les colorants ou les émollients, les cires, les agents nacrants, les charges minérales ou organiques.

Les exemples ci-après illustrent l'invention. Dans ceux-ci, les pourcentages et parties sont pondéraux sauf indication contraire.

### Exemple 1

On mélange sous agitation et sous azote les huiles raffinées ci-après dans les proportions indiquées.

| **Huile** | **%** |
|---|---|
| Huile de tournesol hybride contenant 80 % d'acide oléique en poids des acides gras | 60 |
| Huile de tournesol | 31 |
| Huile de rosa mosqueta | 6 |
| Huile de pépins de cassis | 3 |

Pour ce faire, dans un réacteur en acier inoxydable muni d'un système de double manteau à circulation de fluides pour thermostatisation et d'un agitateur à vitesse variable, on additionne l'huile de pépins de cassis et l'huile de rosa mosqueta aux huiles de tournesol hybride et de tournesol standard en évitant des températures supérieures à 30°C.
On ajoute au mélange un antioxydant, p.ex. la vitamine E (tocopherol et ses esters) ou des antioxydants du type phénolique (p.ex. le butylhydroxyanisole) ou des extraits naturels à pouvoir antioxydant (p.ex. d'épices) dans des proportions allant jusqu'à 1'000 ppm (partie par million) calculées sur le mélange d'huile.
En circuit fermé, on conditionne le mélange en fûts à surface plastifiée, préférablement d'une capacité de 25 kg et sous atmosphère d'azote pour éviter les dégradations oxydatives liées à l'insaturation du mélange.

### Exemple 2

On prépare le mélange d'huiles comme à l'exemple 1, auquel on ajoute sous forme concentrée un acide gras à longue chaîne n-3 pour assurer au mélange un métabolisme optimal sur la peau tenant compte des éventuelles déficiences enzymatiques présentes, par exemple, dans les cas de vieillissement et de desséchement de la peau. Le mélange a les proportions indiquées.

| **Huile** | **%** |
|---|---|
| Huile de tournesol hybride contenant 80 % d'acide oléique en poids des acides gras | 60 |
| Huile de tournesol | 31 |
| Huile de rosa mosqueta | 5,7 |
| Huile de pépins de cassis | 3 |
| Ester éthylique de l'acide éicosapentaénoïque | 0,3 |

### Exemple 3

### Fluide (émulsion huile-dans-l'eau)

| Ingrédient | % |
|---|---|
| Propylène glycol | 2 |
| Peg 400 | 3 |
| Conservateur | 0,3 |
| Carbopol 980 | 0,2 |
| Myristate d'isopropyle | 1 |
| Alcool cétylique et acide stéarique | 3 |
| Monostéarate de glycérol | 3 |
| Huile de germe de maïs | 2 |
| Composition lipidique de l'exemple 1 | 5 |
| Parfum | 0,5 |
| Eau déminéraliséé qsp | 100 |

Pour préparer cette émulsion huile-dans-l'eau, on homogénéise en phase liquide avec un équipement approprié, la phase huileuse contenant la composition lipidique de l'exemple 1, additionnée des autres composants lipidiques et les émulgateurs de la formule, dans la phase aqueuse constituée d'eau déminéralisée et des autres composants hydrophiles tels que conservateurs, parfums, etc.

### Exemple 4

### Huile pour le corps (anhydre)

| Ingrédients | % |
|---|---|
| Composition lipidique de l'exemple 1 | 60 |
| Volatil silicone 7158 | 34,5 |
| Parfum | 0,5 |
| Butanol G | 5 |

### Exemple 5

### Emulsion huile-dans-l'eau

| Ingrédients | % |
|---|---|
| Volatil silicone | 10 |
| Perhydrosqualène | 18 |
| Huile de vaseline | 5 |
| Lanoline liquide | 4 |
| Arlacel 165 (Atlas) | 6 |
| Tween 60 (Atlas) | 2 |
| Alcool cétylique | 1,2 |
| Acide stéarique | 2,5 |
| Triéthanolamine | 0,1 |
| Conservateur | 0,3 |
| Antioxydants | 0,3 |
| Composition lipidique de l'exemple 1 | 10 |
| Eau qsp | 100 |

On dissout à 170°C les composants lipidiques de la formule dans la composition lipidique de l'exemple 1. Après addition des émulsifiants Arlacel 165 et Tween 60, des conservateurs et de l'antioxydant, on émulsifie par homognéisation cette phase huileuse dans la phase aqueuse, qui constitue la phase continue de l'émulsion. On refroidit ensuite l'émulsion à la température ambiante et on la conditionne.

### Exemple 6

### Emulsion eau-dans-l'huile

| Ingrédients | % |
|---|---|
| Protegin X | 20 |
| Huile de vaseline | 5 |
| Glycérine | 5 |
| Sulfate de magnésium | 0,5 |
| Composition lipidique de l'exemple 1 | 25 |
| Composition aromatique | 1 |
| Conservateur | 0,3 |
| Eau qsp | 100 |

La composition lipidique de l'exemple 1, additionnée d'huile de vaseline et de Protegin X constitue la phase continue de l'émulsion obtenue en ajoutant la phase aqueuse contenant la glycérine et le sel de magnesium à la phase lipidique à env. 70°C. Après addition des composants aromatiques et conservateurs de la formule, l'émulsion est homogénéisée, refroidie et conditionnée.

### Exemple 7

### Rouge à lèvres (anhydre)

| Ingrédients | % |
|---|---|
| Composition lipidique de l'exemple 1 | 20 |
| Huile de sésame | 9 |
| Huile de ricin | 15 |
| Huile de vaseline | 15 |
| Lanoline | 15 |
| Cire d'abeilles | 5 |
| Ozokérite | 10 |
| Butylhydroxytoluène | 0,1 |
| Colorants | 5,9 |
| Oxyde de titane | 5 |
| Parfum | qs |

On obtient le produit anhydre précédent de la même manière que dans l'exemple 6, mais sans homogénéisation, par mélange à chaud, puis refroidissement progressif sous brassage lent.

## Revendications

1. Composition lipidique d'application topique pour usage cosmétique, caractérisée par le fait que les acides gras des triglycérides comprennent, en poids, 40 à 70 % d'acide oléique et 30 à 50 % d'acides gras polyinsaturés, que le rapport pondéral des acides gras de la famille n-6 sur ceux de la famille n-3 est 10:1 à 30:1 et que les acides gras polyinsaturés comprennent une quantité efficace d'acides des familles n-3 et n-6 de degré d'insaturation 3 et plus.

2. Composition lipidique selon la revendication 1, caractérisée par le fait qu'elle contient, en poids, 50 à 70 % d'huile de tournesol ou de carthame hybride à haute teneur en acide oléique, 20 à 40 % d'huile de tournesol ou de pépins de raisins, 3 à 10 % d'huile de rosa mosqueta, de chia ou de lin et 1 à 10 % d'huile de pépins de cassis.

3. Composition cosmétique contenant une composition lipidique selon la revendication 1 ou 2.

4. Composition cosmétique selon la revendication 3, caractérisée en ce qu'elle se présente sous forme de solution, d'émulsion eau-dans-l'huile ou huile-dans-l'eau, de suspension ou d'aérosol.

5. Composition cosmétique selon la revendication 3, caractérisée en ce qu'elle se présente sous forme de crème ou de lait.

6. Composition cosmétique selon la revendication 3, caractérisée en ce qu'elle se présente sous forme anhydre, notamment de baume, d'huile pour le corps, d'huile anti-solaire ou de rouge à lèvres.

7. Composition selon l'une des revendications 3 à 5, caractérisée en ce qu'elle contient également au moins un adjuvant cosmétique choisi dans le groupe formé par les émulsifiants, les agents anti-transpirants, les stabilisants, les conservateurs, les antioxydants, les filtres solaires, les parfums, les colorants, les émollients, les agents nacrants, les cires et les charges organiques ou minérales.

## Claims

1. Lipidic composition applicable topically for cosmetic use, characterized in that the fatty acids of the triglycerides comprise, by weight, 40 to 70 % oleic acid and 30 to 50 % of polyunsaturated fatty acids, that the weight ratio of fatty acids of the n-6 family to those of the n-3 family is 10:1 to 30:1 and that the polyunsaturated fatty acids include an effective quantity of acids of the n-3 and n-6 families with a degree of unsaturation of 3 and more.

2. Lipidic composition according to claim 1, characterized in that it contains, by weight, 50 to 70 % of sunflower or hybrid safflower oil with a high oleic acid content, 20 to 40 % sunflower or grape seed oil, 3 to 10 % rosa mosqueta, chia or linseed oil and 1 to 10 % black currant seed oil.

3. Cosmetic composition containing a lipidic composition according to claim 1 or 2.

4. Cosmetic composition according to claim 3, characterized in that it is in the form of a solution, a water-in-oil emulsion or an oil-in-water emulsion, a suspension or an aerosol.

5. Cosmetic composition according to claim 3, characterized in that it is in the form of a cream or milk.

6. Cosmetic composition according to claim 3, characterized in that it is in an anhydrous form, in particular a balm, a body oil, an anti-sun oil or a lipstick.

7. Composition according to one of claims 3 to 5, characterized in that it also contains at least one cosmetic additive chosen from the group formed of emulsifiers, antiperspirant agents, stabilizers, preservatives, anti-oxidants, sun filters, perfumes, colorants, emollients, pearl agents, waxes and organic or inorganic fillers.

## Patentansprüche

1. Lipidzusammensetzung zur topischen Anwendung für eine Verwendung in der Kosmetik, **dadurch gekennzeichnet**, daß die Triglycerid-Fettsäuren, in Gewicht, 40 bis 70% Ölsäure und 30 bis 50% mehrfach ungesättigte Fettsäuren umfassen, wobei das Gewichtsverhältnis der Fettsäuren der Familie n-6 zu dem der Familie n-3 10:1 bis 30:1 beträgt und die mehrfach ungesättigten Fettsäuren eine wirksame Menge von Säuren der Familien n-3 und n-6 mit einem Ungesättigtheitsgrad von 3 und mehr enthalten.

2. Lipidzusammensetzung nach Anspruch 1, dadurch gekennzeichnet, daß sie, in Gewicht, 50 bis 70% Sonnenblumenhybridöl oder Saflorhybridöl mit einem hohen Ölsäuregehalt, 20 bis 40% Sonnenblumenöl oder Traubenkernöl, 3 bis 10% Moschusrosenöl, Chiaöl oder Leinöl und 1 bis 10% Johannisbeerenkernöl enthält.

3. Kosmetische Zusammensetzung, die eine Lipidzusammensetzung nach Anspruch 1 oder 2 enthält.

4. Kosmetische Zusammensetzung nach Anspruch 3, dadurch gekennzeichnet, daß sie in Form einer Lösung, in Form einer Wasser-in-Öl- oder Öl-in-Wasser-Emulsion, einer Suspension oder eines Aerosols vorliegt.

5. Kosmetische Zusammensetzung nach Anspruch 3, dadurch gekennzeichnet, daß sie in Form einer Creme oder einer Milch vorliegt.

6. Kosmetische Zusammensetzung nach Anspruch 3, dadurch gekennzeichnet, daß sie in wasserfreier Form, insbesondere in der eines Balsams, eines Körperöls, eines Sonnenschutzöls oder eines Lippenrots vorliegt.

7. Zusammensetzung nach einem der Ansprüche 3 bis 5, dadurch gekennzeichnet, daß sie auch wenigstens einen kosmetischen Hilfsstoff enthält, der ausgewählt ist aus der Gruppe, die gebildet wird von Emulgatoren, schweißhemmenden Mitteln, Stabilisatoren, Konservierungsmitteln, Antioxidantien, Sonnenlichtfiltermitteln, Parfümen, Farbstoffen, Weichmachern, Perlglanzmitteln, Wachsen und organischen und mineralischen Füllstoffen.
